# EUROPEAN PATENT SPECIFICATION

(11) **EP 1 981 472 B1**
(45) Date of publication and mention of the grant of the patent: **05.08.2009**
(21) Application number: 07703183.9
(22) Date of filing: 25.01.2007
(51) Int. Cl.: A61K 8/73, A61K 8/891, A61K 8/896, A61Q 5/10, A61Q 5/12

(54) **USE OF HAIR TREATMENT COMPOSITIONS FOR CONDITIONING BLEACHED HAIR**
VERWENDUNG VON HAARPFLEGEZUSAMMENSETZUNGEN ZUR KONDITIONIERUNG GEBLEICHTER HAARE
UTILISATION DE COMPOSITIONS CAPILLAIRES POUR CONDITIONNER LES CHEVEUX DÉCOLORÉS

(30) Priority: 08.02.2006 EP 06250677
(43) Date of publication of application: 22.10.2008
(73) Proprietor: Unilever N.V., 3013 AL Rotterdam (NL); Unilever PLC, London EC4Y 0DY (GB)
(72) Inventor: DAWSON, Jayne, Lesley, Merseyside CH63 3JW (GB); DICKINSON, Kelvin, Brian, Merseyside CH63 3JW (GB); EVERAERT, Emmanuel Paul Jos Marie, Merseyside CH63 3JW (GB); HOLT, Neil, Christopher, Merseyside CH63 3JW (GB); SHAW, Neil, Scott, Merseyside CH63 3JW (GB)
(74) Representative: Newbould, Frazer Anthony
(86) International application number: PCT/EP2007/000848
(87) International publication number: WO 2007/090554

(56) References cited:
- EP-A- 0 964 054
- DE-A1- 10 247 695
- DE-A1- 10 260 874
- US-A- 5 877 144
- US-B1- 6 759 052
- KARL BOOTEN, BART LEVECKE: "Inutec: a novel type of polymeric surfactant: Karl Booten and Bart Levecke of Orafti Non-Food describe the fundamental properties of a new type of molecule, with formulations to illustrate their performance" SPECIALITY CHEMICALS, [Online] March 2004 (2004-03), XP002390164 Retrieved from the Internet: URL:http://www.allbusiness.com/periodicals /article/138921-1.html> [retrieved on 2006-07-11]
- AMERICAN CHEMICAL SOCIETY: "Chemical modification of inulin, a valuable renewable resource, and its industrial applications" BIOMACROMOLECULES, [Online] vol. 2, no. 1, 2001, XP002390021 Retrieved from the Internet: URL:http://pubs.acs.org/cgi-bin/article.cg i/bomaf6/2001/2/i01/pdf/bm005642t.pdf> [retrieved on 2006-07-11]

## Description

### Technical Field

The invention is concerned with the use of compositions for conditionning bleached hair. More specifically, it is concerned with improving and targeting the deposition of conditioning oil onto bleached hair from compositions.

### Background and Prior Art

Bleaching the hair in many instances significantly ruins its condition. This can be a problem when hair is highlighted/ streaked as on the same head of hair there are sections of bleached hair that needs intensive conditioning and unbleached hair where the conditioning level should be less intense.

One way of improving conditioning is to increase the deposition of the materials known to condition hair. The use of cationic polymers is one such method of increasing deposition; US Patent 3,753,916 discloses the use of cationic polymers as deposition aids.

The present invention relates to an improved way of conditioning bleached hair. It is particularly useful for bleached hair as the conditioning oil is more substantive to bleached hair than non-bleached hair. One advantage of this technology is that the conditioner can be delivered where it is needed (i.e. on highlighted hair) without adversely affecting hair, which does not require such intensive conditioning.

### Detailed Description of the Invention

The present invention relates to the use of a hair treatment composition comprising:
a) a hydrophobic conditioning oil; and
b) a hydrophobic polysaccharide comprising fructose units, for conditioning bleached hair.

### Hydrophobic Polysaccharide

The composition to be used according to the present invention comprises a hydrophobic polysaccharide comprising fructose units.

It is preferable if the hydrophobic polysaccharide is a straight chain polysaccharide. Suitable polysaccharides are graft copolymers, it is especially preferred if the polymer comprises an alkyl chain having from 10 to 22 carbon atoms.

Preferably the hydrophobic polysaccharide has a molecular weight less than 20,000 g.mol⁻¹ more preferably from 600 g.mol⁻¹ to 10,000 g.mol⁻¹, most preferably from 2,000 to 6,000 g.mol⁻¹.

Suitable polymers for use with the invention are disclosed in Langmuir 2003, 19, 10463-10467.

A particularly preferred hydrophobic polysaccharide is inulin lauryl carbamate (lauramidoyl inulin) such as Inutec SP1 ex Orafti.

Preferably the level of hydrophobic polysaccharide is from 0.01 to 10 wt% of the total composition, more preferably from 0.1 to 5 wt %'of the total composition.

### Hydrophobic Conditioning Oil

Compositions to be used according to the invention comprise a water-insoluble hydrophobic conditioning oil. This may be a non-silicone hydrophobic oil but is more preferably a silicone conditioning agent. By water insoluble it is meant that the material has a solubility in water of 0.1% or less by weight of water at 25°C. Preferably the conditioning agent is non-volatile, meaning that it has a vapour pressure of less than 1000 Pa at 25°C. The conditioning oil is present in the composition as discrete emulsion droplets.

Emulsified hydrophobic conditioning oils for use in the shampoo or shower gel compositions of the invention suitably have an average droplet diameter (D_{3,2}) in the composition of 4 micrometres or less, preferably 2 micrometres or less, more preferably 1 micrometre or less.

A suitable method for measuring the D_{3,2} mean diameter is by laser light scattering using an instrument such as a Malvern Mastersizer.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the silicone itself (not the emulsion or the final hair conditioning composition) is typically from 350 to 200,000,000 mm²sec⁻¹ at 25°C. Preferably the viscosity is at least 5,000 mm² sec ⁻¹ at 25°C, more preferably at least 10,000 mm²sec⁻¹. Preferably the viscosity does not exceed 20,000,000 mm²sec⁻¹, more preferably 10,000,000 mm²sec⁻¹, most preferably 5,000,000 mm²sec⁻¹.

Viscosity of silicones can be measured using a glass capillary viscometer as set out in Dow Corning corporate test method CTM004 July 20, 1970 at 25°C. Viscosities are generally provided by suppliers of silicones, either as measured or as dedeuced from their molecular weight.

In some instances the silicone oil also comprises a functionalised silicone. Suitable functionalised silicones include, amino-, carboxy-, betaine-, quaternary ammonium-, carbohydrate-, hydroxy- and alkoxy-substituted silicones. Preferably, the functionalised silicone contains multiple substitutions.

For the avoidance of doubt, as regards hydroxyl-substituted silicones, a polydimethylsiloxane merely having hydroxyl end groups (which have the CTFA designation dimethiconol) is not considered a functionalised silicone within the definition of the present invention. However, a polydimethylsiloxane having hydroxyl substitutions along the polymer chain is considered a functionalised silicone.

A class of functionalised silicone for inclusion in compositions of the invention is amino functional silicone. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones). Suitable quaternary silicone polymers are described in EP-A-0 530 974. A preferred quaternary silicone polymer is K3474, ex Goldschmidt.

Another preferred functional silicone for use as a component in the hydrophobic conditioning oil is an alkoxy-substituted silicone. Such molecules are known as silicone copolyols and have one or more polyethyleneoxide or polypropyleneoxide groups bonded to the silicone polymer backbone, optionally through an alkyl linking group.

Suitable silicone copolyols have an HLB of 10 or less, preferably 7 or less, more preferably 4 or less. A suitable silicone copolyol material is DC5200, known as Lauryl PEG/PPG - 18/18 methicone (INCI name), available from Dow Corning.

Hydrophile/Lipophile balance or HLB is a well known parameter used by those skilled in the art to characterise surface active molecules and emulsifiers. Suitable methods for the experimental determination of HLB are in Griffin W.C, Journal of the Society of Cosmetic Chemists, volume 1 page 311 (1949). The commercially available silicone copolyols are supplied along with a value of their HLB by Dow Corning.

Combination silicones may be used.

The total amount of silicone is preferably from 0.01% to 10 % by weight of the total composition more preferably from 0.1% to 5%, most preferably 0.5% to 3%.

The silicones may be added to the composition as a fluid and subsequently emulsified, but preferably are added as preformed emulsions. More preferably, the silicone emulsions additionally comprise the hydrophobic polysaccharide, such as Inutec SP1, prior to addition to the remainder of the product.

### Non-silicone Hydrophobic Conditioning Oil

Compositions to be used according to the present invention may comprise a dispersed, non-volatile, water-insoluble oily non-silicone conditioning agent.

Suitable oily or fatty materials are selected from hydrocarbon oils, fatty esters and mixtures thereof.

Straight chain hydrocarbon oils will preferably contain from about 12 to about 30 carbon atoms. Also suitable are branched chain hydrocarbon oils will preferably contain from about 12 to about 42 carbon atoms. Also suitable are polymeric hydrocarbons of alkenyl monomers, such as C2-C6 alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Another suitable material is polyisobutylene.

Suitable fatty esters are characterised by having at least 10 carbon atoms, and include esters with hydrocarbyl chains derived from fatty acids or alcohols, Monocarboxylic acid esters include esters of alcohols and/or acids of the formula R' COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and triesters of glycerol and long chain carboxylic acids such as C1-C22 carboxylic acids. Preferred materials include cocoa butter, palm stearin, sunflower oil, soyabean oil and coconut oil.

Preferably, the viscosity of the conditioning oil itself (not the emulsion or the final hair conditioning composition) is from 350 to 10, 000, 000 mm²sec⁻¹ at 25°C. More preferably the viscosity is at least 5,000 mm²sec⁻¹ at 25 °C, most preferably at least 10,000 mm²sec⁻¹. Preferably the viscosity does not exceed 500,000 mm²sec⁻¹.

The oily or fatty material is suitably present at a level of from 0.05 to 20, preferably from 0.2 to 10, more preferably from about 0.5 to 5 percent by weight of the composition.

### Aqueous Conditioning Composition.

The compositions to be used according to the invention are preferably rinse off and/or aqueous. By aqueous conditioning composition is meant a composition which has water or an aqueous solution or a lyotropic liquid crystalline phase as its major component. Suitably, the composition will comprise from 50% to 98% by weight of water, preferably from 60% to 90%. Particularly preferred manifestations of the invention are hair shampoos and/or hair conditioners. Hair shampoos comprise cleansing surfactants and may further comprise cationic deposition polymers, suspending agents and adjuvants. Hair conditioners comprise conditioning surfactants.

### Cleansing Surfactant

Shampoo compositions to be used according to the invention will comprise one or more cleansing surfactants, which are cosmetically acceptable and suitable for topical application to the hair. Further surfactants may be present as an additional ingredient if sufficient for cleansing purposes is not provided by the emulsifier for the water-insoluble oily component. It is preferred that the shampoo compositions comprise at least one further surfactant (in addition to that used as emulsifying agent for the water-insoluble oily component) to provide a cleansing benefit. Suitable cleansing surfactants, which may be used singularly or in combination, are selected from anionic, amphoteric and zwitterionic surfactants and mixtures thereof. The cleansing surfactant may be the same surfactant as the emulsifier, or may be different.

### Anionic Cleansing Surfactant

The shampoo compositions will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl-ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n is from 1 to 3), ammonium lauryl sulphate and ammonium lauryl ether sulphate(n)EO, (where n is from 1 to 3).

Mixtures of any of the foregoing anionic cleansing surfactants may also be suitable.

The total amount of anionic cleansing surfactant in shampoo compositions of the invention is generally from generally from 0.5 to 45, preferably from 1.5 to 35, more preferably from 5 to 20 percent by weight of the composition.

### Co-surfactant

The composition can include co-surfactants, to help impart aesthetic, physical or cleansing properties to the composition.

A preferred example is an amphoteric or zwitterionic surfactant, which can be included in an amount ranging from 0 to about 8, preferably from 1 to 4 percent by weight of the composition.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred example is a nonionic surfactant, which can be included in an amount ranging from 0 to 8, preferably from 2 to 5 percent by weight of the composition. For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The composition to be used according to the invention can also optionally include one or more cationic co-surfactants included in an amount ranging from 0.01 to 10, more preferably from 0.05 to 5, most preferably from 0.05 to 2 percent by weight of the composition.

The total amount of surfactant (including any co-surfactant, and/or any emulsifier) in compositions of the invention is generally from 1 to 50, preferably from 2 to 40, more preferably from 10 to 25 percent by weight of composition.

A preferred blend of cleansing surfactants is a combination of ammonium lauryl ether sulphate, ammonium lauryl sulphate, PEG 5 cocamide and cocamide MEA (CTFA designations).

### Cationic Deposition Polymer

A cationic polymer may be present for further enhancing conditioning performance of the shampoo.

The cationic polymer may be a homopolymer or be formed from two or more types of monomers. The molecular weight of the polymer will generally be between 5 000 and 10 000 000 Dalton, typically at least 10 000 and preferably from 100 000 to 2 000 000 Dalton. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl, and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic conditioning polymers can comprise mixtures of monomer units derived from amine- and/or quaternary ammonium-substituted monomer and/or compatible spacer monomers.

Suitable cationic conditioning polymers include, for example:
- copolymers of 1-Vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311).

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density from 0.1 to 4 meq/g.

Cationic polysaccharide polymers suitable for use in compositions of the invention include those of the formula:

A-O-[R-N⁺ (R¹) (R²) (R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic are Hydrophobically modified cationic cellulose, INCI polyquaternium 67, as described in WO 2005/000903 A1 from Amerchol corp. Preferred PQ67 are available from Amerchol corp under Tradename SofCat.

Other suitable cationic polysaccharide polymers include quaternary nitrogen-containing cellulose ethers (e.g. as described in U.S. Patent 3,962,418), and copolymers of etherified cellulose and starch (e.g. as described in U.S.. Patent 3, 958, 581).

A particularly suitable type of cationic polysaccharide polymer that can be used is a cationic guar gum derivative, such as guar hydroxypropyltrimonium chloride (commercially available from Rhodia in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic conditioning polymer will generally be present in compositions of the invention at levels of from 0.01 to 5, preferably from 0.02 tol 1, more preferably from 0.04 to 0.5 percent by weight of the composition.

### Suspending Agents

Optionally, the compositions to be used according to the invention further comprise from 0.1 to 10 percent by weight, preferably from 0.6% to 6%, of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

### Adjuvants

The compositions to be used according to the present invention may also contain adjuvants suitable for hair care. Generally such ingredients are included individually at a level of up to 2 percent by weight of the total composition.

Among suitable hair care adjuvants, are natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine. Another suitable adjuvant is glycolic acid.

### Mode of Use

The compositions previously described are for topical application to the hair and/or scalp of a human subject and are preferably rinse-off compositions.

The invention is further demonstrated with reference to the following, non-limiting examples:
Examples of the invention are illustrated by a number, comparative Examples are illustrated by a letter.

### Examples

### Improved silicone deposition on bleached hair using hydrophobised inulin

| | | **Wt %** | |
|---|---|---|---|
| | | **Example 1** | **Example A** |
| Sodium laureth sulphate 1EO) | (Texapon N701 ex Cognis | 6.0 | 6.0 |
| Disodium laureth sulphosuccinate | Mackanate EL ex Mackintye | 4.0 | 4.0 |
| Cocoamidopropyl betaine | (Tegobetaine CK ex Goldschmidt) | 3 | 3 |
| Carbomer | (Carbopol 980 ex Goodrich) | 0.4 | 0.4 |
| Guar hydroxypropyl trimonium chloride | Jaguar C13S Ex Rhodia | 0.2 | 0.2 |
| Polydimethyl siloxane¹ | DC-1786 | 5 | 5 |
| Inulin lauryl carbamate. | Inutec SP1 Ex Orafti | 0.5 | |
| NaCl | | 2.0 | 2.0 |
| Water | | to 100 | to 100 |

A silicone emulsion, DC-1786 from Dow Corning, (comprising droplets of a high viscosity (1McS) polydimethylsiloxane polymer with mean droplet diameter approximately 250nm, emulsified with triethanolamine dodecyl benzene, prepared by an emulsion-polymerisation route)was mixed with an equal mass of a 10% aqueous suspension of a hydrophobised inulin (Inutec SP1 from Orafti). This mixture is hereafter referred to as Silicone Emulsion 1A.

Guar hydroxypropyl trimonium chloride was prepared as a 1% active aqueous and the carbomer as a 4% active aqueous suspension. The shampoo formulations were then prepared by combining all the ingredients (pre-mixes) to distilled water in an appropriate vessel and mixing at ambient temperature using an overhead paddle stirrer. The sodium chloride should be the final ingredient to be added.

The Examples were each used to wash switches of human hair. The washing was done by weighing out a fixed mass of shampoo (0.1g shampoo per 1g of hair to be washed), applying to the hair and adding a fixed mass of water (1g water per 1g hair) before lathering for 30s and rinsing for 30s. The washed hair switches were allowed to dry before being analysed for deposited silicone by X-ray fluorescence spectrometry. For each prototype, this experiment was performed on both virgin dark brown European hair and also on hair from the same source which had been bleached using a commercial hair bleaching product. Each experiment was performed on 5 replicate switches. The mean silicone deposition data (normalised by the silicone deposition of Example 1 on virgin hair) can be seen below:

| | **Normalised silicone deposition on virgin hair (+/-0.1)** | **Normalised silicone deposition on bleached hair (+/- 0.1)** | **Ratio (Bleached / Normal)** |
|---|---|---|---|
| Example A | 1.00 | 0.74 | 0.74 |
| Example 1 | 1.01 | 1.09 | 1.08 |

Example 1, which is according to the invention, shows superior silicone deposition on bleached hair in comparison with comparative Example A, which is not according to the invention. Furthermore, the deposition of silicone on virgin hair is comparable for the two prototypes, so Example 1 displays superior targeting of silicone deposition towards bleached hair.

### Improved silicone deposition on bleached hair using hydrophobised inulin - further evidence

### Shampoo Formulation

| | | **Wt %** | | |
|---|---|---|---|---|
| | | **Example 2** | **Example B** | **Example C** |
| Sodium laureth sulphate 1EO) | (Texapon N701 ex Cognis | 12 | 12 | 12 |
| Cocoamidopropyl betaine | (Tegobetaine CK ex Goldschmidt) | 1.6 | 1.6 | 1.6 |
| Carbomer | (Carbopol 980 ex Goodrich) | 0.4 | 0.4 | 0.4 |
| Guar hydroxypropyl trimonium chloride | Jaguar C13S Ex Rhodia | 0.2 | 0.2 | 0.2 |
| Polydimethyl siloxane¹ | DC-1786 | 5 | 0.5 | 5 |
| Inulin lauryl carbamate. | Inutec SP1 Ex Orafti | 0.5 | | |
| non-hydrophobised Inulin | Inutec H25P Ex Orafti | | 0.5 | |
| Poloxamer 217 | Pluronic F77 Ex BASF | | | 0.5 |
| NaCl | | 0.75 | 0.75 | 0.75 |
| Water | | to 100 | to 100 | to 100 |

1 is a high viscosity (1McS) polydimethylsiloxane polymer with mean droplet diameter approximately 250nm.

A pre-emulsion was prepared by adding DC-1786 from Dow Corning, with either hydrophobised inulin (Inutec SP1 from Orafti) to give Example 2, unmodified inulin (Inutec H25P from Orafti) Example B or Poloxamer 217 (Pluronic F77 from BASF) Example C. These pre-emulsions were added to a shampoo formulation as described previously.

The Examples were each used to wash switches of human hair. The washing was done by weighing out a fixed mass of shampoo (0.1g shampoo per 1g of hair to be washed), applying to the hair and adding a fixed mass of water (lg water per 1g hair) before lathering for 30s and rinsing for 30s. The washed hair switches were allowed to dry before being analysed for deposited silicone by X-ray fluorescence spectrometry. For each prototype, this experiment was performed on both virgin dark brown European hair and also on hair from the same source, which had been bleached using a commercial hair-bleaching product. Each experiment was performed on 5 replicate switches. The mean silicone deposition data (normalised by the silicone deposition of Prototype C on virgin hair) can be seen below:

| | **Normalised silicone deposition on virgin hair (+/-0.1)** | **Normalised silicone deposition on bleached hair (+/-0.1)** | **Ratio (Bleached / Normal)** |
|---|---|---|---|
| Example 2 | 1.00 | 1.17 | 1.17 |
| Example B | 1.06 | 0.98 | 0.92 |
| Example C | 0.87 | 0.92 | 1.06 |

| | | | |
|---|---|---|---|
| Example 2, which is according to the invention, shows superior silicone deposition on bleached hair in comparison with Examples B and C, which are not according to the invention. Furthermore, Example 2 displays substantially superior targeting of silicone deposition towards bleached hair in comparison with Example B and directionally superior targeting of silicone deposition towards bleached hair in comparison with Example C. | | | |

### Shampoo Formulation

| **Trade Name** | **Chemical Name** | **Example 3** |
|---|---|---|
| Texapon N701 | SodiumLaurylEtherSulfate 1-EO | 8.0 |
| Tegobetain CK | Cocoamidopropylbetaine | 2.0 |
| Carbopol 980 | Carboxymethylcellulose | 0.4 |
| Silicone DC1786 | Polydimethylsiloxane | 10.0 |
| Inutec SP1 | Inulin lauryl carbamate. | 1.0 |
| | Water and minors | To 100% |

### Conditioner Formulation

| **Ingredient** | **Example 4** |
|---|---|
| Cetyl trimethyl ammonium chloride (29% active) | 2.4 |
| Cetearyl alcohol | 2.1 |
| NATROSOL® 250HHR⁽¹⁾ | 1.0 |
| Inutec SP1 | 1.0 |
| Silicone DC1786 | 10.0 |
| Phenoxyethanol | 0.4 |
| Water and minors | to 100 |

| | |
|---|---|
| ⁽¹⁾ Hydroxyethylcellulose, ex Aqualon. | |

## Claims

1. Use of a hair treatment composition comprising:
a) a hydrophobic conditioning oil; and
b) a hydrophobic polysaccharide comprising fructose units, for conditioning bleached hair.

2. Use according to claim 1 which the hydrophobic polysaccharide is a straight chain polysaccharide.

3. Use according to claim 1 or 2 in which the hydrophobic polysaccharide further comprises an alkyl group having from 10 to 22 carbon atoms.

4. Use according to any preceding claim in which the hydrophobic polysaccharide is inulin lauryl carbamate.

5. Use according to any preceding claim in which the level of hydrophobic polysaccharide in the total composition is from 0.01 to 10 wt%

6. Use according to any preceding claim wherein the hydrophobic conditioning oil is a silicone oil.

7. Use according to any preceding claim wherein the composition is formulated as a rinse off product.

8. Use according to any preceding claim that is an aqueous based shampoo.

9. Use according to any claim 8 comprising from 2 to 40 percent by weight of a cleansing surfactant selected from the group consisting of anionic surfactants; nonionic surfactants, amphoteric surfactants and mixtures thereof.

10. Use according to claim 8 or claim 9 wherein the composition further comprises a cationic deposition polymer.

11. Use according to any one of claims 8 to 10 wherein the cationic deposition polymer is a cationic derivative of guar gum.

12. Use according to any preceding claim in which the hydrophobic conditioning oil has a mean diameter (D_{3,2}) of 4 micrometres or less.

13. Use according to claim 12 wherein the mean diameter of the droplets of hydrophobic conditioning oil (D_{3,2}) is 1 micrometre or less.

## Patentansprüche

1. Verwendung einer Haarbehandlungszusammensetzung, umfassend:
a) ein hydrophobes konditionierendes Öl und
b) ein hydrophobes Polysaccharid, das Fructoseeinheiten umfasst, zur Konditionierung von gebleichtem Haar.

2. Verwendung gemäß Anspruch 1, wobei das hydrophobe Polysaccharid ein geradkettiges Polysaccharid ist.

3. Verwendung gemäß Anspruch 1 oder 2, wobei das hydrophobe Polysaccharid außerdem eine Alkylgruppe mit 10 bis 22 Kohlenstoffatomen umfasst.

4. Verwendung gemäß einem vorangehenden Anspruch, wobei das hydrophobe Polysaccharid Inulinlaurylcarbamat ist.

5. Verwendung gemäß einem vorangehenden Anspruch, wobei die Konzentration an hydrophobem Polysaccharid in der Gesamtzusammensetzung 0,01 bis 10 Gewichts-% ist.

6. Verwendung gemäß einem vorangehenden Anspruch, wobei das hydrophobe konditionierende Öl ein Silikonöl ist.

7. Verwendung gemäß einem vorangehenden Anspruch, wobei die Zusammensetzung als ein Produkt zum Ausspülen formuliert ist.

8. Verwendung gemäß einem vorangehenden Anspruch, wobei es sich um ein Shampoo auf wässriger Basis handelt.

9. Verwendung gemäß Anspruch 8, außerdem umfassend 2 bis 40 Gewichts-% eines Reinigungs-Tensids, ausgewählt aus der Gruppe, bestehend aus anionischen Tensiden, nicht-ionischen Tensiden, amphoteren Tensiden und Gemischen davon.

10. Verwendung gemäß Anspruch 8 oder Anspruch 9, wobei die Zusammensetzung außerdem ein kationisches Depositionspolymer umfasst.

11. Verwendung gemäß einem der Ansprüche 8 bis 10, wobei das kationische Depositionspolymer ein kationisches Derivat von Guargummi ist.

12. Verwendungszusammensetzung gemäß einem vorangehenden Anspruch, wobei das hydrophobe konditionierende Öl einen mittleren Durchmesser (D_{3,2}) von 4 Mikrometern oder weniger hat.

13. Verwendungszusammensetzung gemäß Anspruch 12, wobei der mittlere Durchmesser der Tröpfchen des hydrophoben konditionierenden Öls (D_{3,2}) 1 Mikrometer oder weniger ist.

## Revendications

1. Utilisation d'une composition de traitement capillaire comprenant :
a) une huile de conditionnement hydrophobe ; et
b) un polysaccharide hydrophobe comprenant des motifs de fructose, pour le traitement des cheveux décolorés.

2. Utilisation selon la revendication 1, dans laquelle le polysaccharide hydrophobe est un polysaccharide à chaîne linéaire.

3. Utilisation selon la revendication 1 ou 2, dans laquelle le polysaccharide hydrophobe comprend en outre un groupe alkyle ayant de 10 à 22 atomes de carbone.

4. Utilisation selon l'une quelconque des revendications précédentes, dans laquelle le polysaccharide hydrophobe est le laurylcarbamate d'inuline.

5. Utilisation selon l'une quelconque des revendications précédentes, dans laquelle la teneur en polysaccharide hydrophobe dans la composition totale est de 0,01 à 10 % en poids.

6. Utilisation selon l'une quelconque des revendications précédentes, dans laquelle l'huile de conditionnement hydrophobe est une huile de silicone.

7. Utilisation selon l'une quelconque des revendications précédentes, dans laquelle la composition est formulée sous la forme d'un produit à rincer.

8. Utilisation selon l'une quelconque des revendications précédentes, qui est un shampooing à base d'eau.

9. Utilisation selon la revendication 8, comprenant de 2 à 40 % en poids d'un tensioactif nettoyant choisi dans le groupe comprenant les tensioactifs anioniques, les tensioactifs non ioniques, les tensioactifs amphotères et leurs mélanges.

10. Utilisation selon la revendication 8 ou 9, dans laquelle la composition comprend en outre un polymère de dépôt cationique.

11. Utilisation selon l'une quelconque des revendications 8 à 10, dans laquelle le polymère de dépôt cationique est un dérivé cationique de la gomme de guar.

12. Utilisation selon l'une quelconque des revendications précédentes, dans laquelle l'huile de conditionnement hydrophobe a un diamètre moyen (D_{3,2}) de 4 micromètres ou moins.

13. Utilisation selon la revendication 12, dans laquelle le diamètre moyen des gouttelettes d'huile de conditionnement hydrophobe (D_{3,2}) est de 1 micromètre ou moins.
